## Europäisches Patentamt

## European Patent Office

## Office européen des brevets

(19)

(11) Publication number: **0 046 397**
B1

# EUROPEAN PATENT SPECIFICATION

(12)

(45) Date of publication of patent specification: 25.04.84

(21) Application number: 81303736.3

(22) Date of filing: 17.08.81

(51) Int. Cl.³: **C 07 C 63/313,**
C 07 C 51/21,
C 07 C 51/235 //B01J27/06,
B01J23/84

(54) Process for producing trimellitic acid or pyromellitic acid.

(30) Priority: 18.08.80 JP 113249/80

(43) Date of publication of application:
24.02.82 Bulletin 82/8

(45) Publication of the grant of the patent:
25.04.84 Bulletin 84/17

(84) Designated Contracting States:
BE DE FR GB

(56) References cited:
CS - B - 175 606
GB - A - 1 342 961
US - A - 3 507 914

Chemical Abstracts vol. 78, no. 23, 11 June
1973 Columbus, Ohio, USA Y. FUJITA et al.
"Aromatic carboxylic acids" page 347, abstract
no. 147581j

(73) Proprietor: MITSUBISHI GAS CHEMICAL
COMPANY, INC.
5-2, Marunouchi 2-chome Chiyoda-Ku
Tokyo (JP)

(72) Inventor: Suzuki, Takashi
4248, Tsukefunecho-1-chome
Niigata-shi (JP)
Inventor: Kitahara, Kouichi
13-31, Yuraku-1-chome
Niigata-shi (JP)
Inventor: Naito, Susumu
5929, Ikarashi-1-chome
Niigata-shi (JP)

(74) Representative: Baillie, Iain Cameron et al,
c/o Langner Parry Isartorplatz 5
D-8000 München 2 (DE)

Courier Press, Leamington Spa, England.

Process for producing trimellitic acid or pyromellitic acid

This invention relates to a process for producing trimellitic acid or pyromellitic acid by the oxidation of at least one of 2,4-dimethylbenzaldehyde, 3,4-dimethylbenzaldehyde and 2,5-dimethylbenzaldehyde or (hereinafter referred to briefly as 2,4-DMBAL, 3,4-DMBAL and 2,5-DMBAL, respectively, and these aldehydes are referred to generically as polymethylated aromatic aldehyde).

Aromatic polycarboxylic acids have conventionally been produced by the oxidation of polyalkylbenzenes and it is known that trimellitic acid is produced from pseudocumene, trimesic acid from mesitylene, pyromellitic acid from durene, and mellophanic acid from isodurene.

These aromatic polycarboxylic acids are used as raw materials for high-grade plasticizers and, not only that, trimellitic acid or pyromellitic acid is used as a raw material for heat-resistant plastics such as polyimideamide or polyimide resins.

Polymethylbenzenes used as starting materials in producing aromatic carboxylic acids are separated by distillation from a catalytic reformate or a cracking residue. Further, polymethylbenzenes synthesized by the methylation or disproportionation of lower methylbenzenes are used likewise.

In recent years, however, as it became practicable to carry out on commercial scale the Gattermann-Koch reaction, whereby an aromatic aldehyde is produced by the formylation of an aromatic hydrocarbon, the route of preparing an aromatic polycarboxylic acid starting from the polyalkylated aromatic aldehyde in place of a polyalkylbenzene has become important.

For the liquid phase oxidation of a polyalkylbenzene, there have been known a method employing nitric acid and another method which utilizes an air oxidation technique. The latter method is widely used, the most typical example being the production of terephthalic acid from p-xylene in acetic acid as solvent with a catalyst system comprising a heavy metal salt and bromine.

British Patent 1 342 961 discloses a process for reacting a tetraalkylbenzene, or derivative thereof such as a monoformyltrimethylbenzene, with molecular oxygen in a medium of a $C_2$ to $C_8$ carboxylic acid in the presence of a catalyst consisting of a heavy metal and bromine at temperatures from 95° to 240°C.

The oxidation of polyalkylbenzenes to aromatic polycarboxylic acids is not always practicable under the same conditions. It is known that the reactivity of a polyalkylbenzene varies depending upon the position of substituent methyl group. In the case of pseudocumene or durene, the trimellitic acid or pyromellitic acid which is formed has two carboxyl groups in the ortho position and such a structure results in a decreased oxidation yield, as compared with the oxidation of other polymethylbenzenes having other structures. To avoid the difficulty, various proposals had been reported to improve the performance of catalyst system. Although being effective for the improvement of material yield, the proposed catalyst systems were of complex compositions which made difficult the recovery and reuse of the catalyst components.

Under the circumstances, the present inventors proposed an improved process wherein the oxidation of pseudocumene was carried out at two stages with different temperature ranges, while maintaining the heavy metal concentration within specified ranges. Although this process was successful in improving the oxidation yield and recovering the catalyst, yet there remained a disadvantage of complication originated from the two-stage reaction.

On the other hand, the techniques for the oxidation of aromatic aldehydes to aromatic polycarboxylic acids is not yet established, except for only one known case of oxidizing p-toluary to terephthalic acid [British Patent Nos. 1518881 and 1518901]. These patents disclose that in producing terephthalic acid by the oxidation of p-tolualdehyde, various difficulties are encountered if the same reaction conditions as those suitable for producing terephthalic acid by the oxidation of p-xylene are employed and, hence, it is necessary to adopt such means as keeping the water content of the reaction system below a certain level or decreasing the concentration of manganese in the catalyst system below a certain value. It is thus necessary to select adequately the reaction conditions and the manner in which the reaction is carried out, depending on the position of substituent and the type of substituent, that is, whether it is an alkyl group or aldehyde group. The present inventors conducted extensive studies taking the above facts into consideration and, as a result, accomplished the present invention.

The present invention provides a process for producing trimellitic acid or pyromellitic acid by the liquid phase air oxidation of at least one of 2,4-DMBAL, 3,4-DMBAL and 2,5-DMBAL in acetic acid as solvent in the presence of cobalt, manganese and bromine as catalyst, which is characterized by carrying out the oxidation in a reactor of complete mixing type at a total concentration of cobalt and manganese of 0.05 to 0.5% by weight based on acetic acid and a reaction temperature of 180° to 240°C.

According to this invention, it is possible to obtain very easily and in high yield an aromatic polyoxycarboxylic acid from a polymethylated aromatic aldehyde in a single reactor and in a continuous way.

The polymethylated aromatic aldehydes used as starting materials in the process of this invention are 2,4-DMBAL, 2,3-DMBAL, and 2,5-DMBAL, which are obtained by the formylation of aromatic hydrocarbons, i.e. xylenes and pseudocumene, with a catalyst such as, for example, the combination of hydrogen fluoride and boron trifluoride, 2,4-DMBAL, 2,3-DMBAL and 2,5-DMBAL can be used alone or in mixture as a raw material for trimellitic acid.

The catalyst system used in the present process is that comprising a combination of two heavy metals of cobalt and manganese with bromine. The total amount of cobalt and manganese should be in the range of from 0.05 to 0.5% by weight based on acetic acid used as solvent. If the total amount of heavy metals is below the lower limit, the smooth progress of the reaction is hindered and the steady oxidation becomes difficult, while nothing is gained from the addition of catalyst in excess of the upper limit, because the excess of catalyst has no effect in favor of the normal reaction but promotes the combustion of the solvent.

The atomic ratio between cobalt and manganese is suitably in the range of from 1:6 to 4:1. The atomic ratio of bromine to the total of cobalt and manganese should preferably be from 0.8 to 2.5,, most preferably 2.0.

Cobalt and manganese can be used in the form of organic acid salts such as acetate propionate, naphthenate, octanate etc., inorganic acid salts such as halides, borates, nitrates, etc. or organic complexes. Bromine can be used in the form of not only simple bromine, but also inorganic bromides, hydrobromic acid and organic bromides. Cobalt bromide and manganese bromide are very suitable, as they contain two of the catalyst components in one molecule.

The mother liquor or catalyst components obtained on separating the product carboxylic acid can be recycled to the oxidation step for reuse after dehydration, purification or concentration, if necessary.

The solvent for use in the present process is acetic acid. Other aliphatic carboxylic acids may be used but are of no practical merit. The acetic acid for use in the process becomes more suitable with the decrease in water content. However, 95% acetic acid (5% in water content) is most suitable for use on commercial scale, when the cost of dehydrating acetic acid is taken into account.

The amount to be used of the solvent, i.e. acetic acid, is preferably 1.0 to 5.0 times the weight of polymethylated aromatic aldehyde used as starting material in the feed for oxidation step. To increase the space time yield of the reactor, a most preferred range is from 2.0 to 3.0 times. If the amount is below the lower limit, the steady progress of the reaction will be hindered or the formation of slurry of the reaction product will become impossible, whereas if the amount exceeds the upper limit, the space time yield will be decreased to render the process uneconomical.

The oxidation temperature is in the range of from 180° to 240°C, preferably from 190° to 230°C. The operation at other temperatures results in an increase in by-products and a decrease in the yield of intended acid.

The reaction pressure is normally sufficient for maintaining the reaction system in liquid state and may be selected from the range of from 588 to 2942 K Pa (6 to 30 kg/cm$^2$) gauge.

The residence time [(liquid holdup in reactor]/(liquid flow)] of the reacting mixture in the oxidation step is not predeterminable unconditionally, because it varies with the reaction conditions. It is generally in the range of from 1 to 5 hours.

The complete-mixing type of reactor used in the present process should preferably be a tank-type provided with a stirrer and an air inlet nozzle, which is commonly used in the liquid phase oxidation.

The invention is further illustrated below with reference to Examples.

### Examples 1 to 4

Oxidation experiments were conducted in a 2-liter oxidation reactor made of titanium having an inner diameter of 56 mm.

The reactor was equipped with a reflux condensor, a feeding nozzle for the reactant mixture, an air inlet nozzle, and a nozzle for withdrawing the contents. It was stirred by means of a rotating agitator provided with four turbine blades.

The reactant mixture was charged into the reactor and after the temperature had been elevated the air supply was turned on to start the reaction. Thereafter, feeding of the reactant mixture was continued at a predetermined rate and the air was supplied continuously. The reaction mixture was withdrawn at a rate so as to maintain the surface of reacting mixture in the reactor at a constant level. After the composition of the reaction mixture had become constant, samples were collected for analysis.

The experimental results and the reaction conditions were as shown in Table.

Cobalt, manganese and bromine were charged in the form of cobalt acetate, manganese acetate and hydrogen bromide respectively.

TABLE

| | Example 1 | Example 2 | Example 3 |
|---|---|---|---|
| Feed flow and catalyst | 2,4-DMBAL | 3,4-DMBAL | Mixture** |
| Aromatic aldehyde (g/hours) | 156 | 160 | 150 |
| Acetic acid (g/hour) | 368 | 370 | 380 |
| Water (g/hour) | 19 | 20 | 20 |
| CO* (ppm) | 390 | 800 | 400 |
| Mn* (ppm) | 1170 | 800 | 1170 |
| Br* (ppm) | 4460 | 4400 | 4400 |
| Reaction conditions | | | |
| Temperature (°C) | 220 | 220 | 220 |
| Pressure (K Pa) | 2157 | 2157 | 2157 |
| Residence time (hour) | 2.1 | 2.1 | 2.5 |
| Results | | | |
| Exhaust gas flow (l/hour) | 584 | 600 | 600 |
| $CO_x$ formation (mole/mole aldehyde) | 2.1 | 2.2 | 2.3 |
| $O_2$ absorption (mole/mole aldehyde) | 4.7 | 4.8 | 4.9 |
| Starting material (mole%) | 0 | 0 | 0 |
| Monocarboxylic acid (mole%) | 0.3 | 1.0 | 0 |
| Dicarboxylic acid (mole%) | 2.6 | 3.0 | 2.0 |
| Tricarboxylic acid (mole%) | 78.7 | 81.0 | 82.0 |
| Tetracarboxylic acid (mole%) | — | — | — |

Note: * Concentration based on acetic acid.
** 1:1 mixture of 2,4-DMBAL and 3,4-DMBAL.

## Claims

1. A process for producing trimellitic acid or pyromellitic acid by liquid phase air oxidation of at least one 2,4-dimethylbenzaldehyde, 3,4-dimethylbenzaldehyde and 2,5-dimethylbenzaldehyde in acetic acid as a solvent in the presence of cobalt, manganese, and bromine as a catalyst, wherein the improvement comprises carrying out the oxidation in a reactor of complete mixing type at a total concentration of the cobalt and the manganese of 0.05—0.5% by weight on the basis of the acetic acid and a reaction temperature of 180°—240°C.

2. A process according to claim 1, wherein an atomic ratio of the cobalt to the manganese is 1:6—4:1 and an atomic ratio of the bromine to total of the cobalt and the manganese is 0.8:1—2.5:1.

3. A process according to Claim 2, wherein the atomic ratio of the bromine to the total of the cobalt and the manganese is 2.0:1.

4. A process according to Claim 1, wherein the cobalt and the manganese are in the form of organic acid salts, inorganic acid salts or organic complexes and the bromine is in the form of simple bromine, inorganic bromides or organic bromides.

5. A process according to Claim 1, wherein the acetic acid is 95% acetic acid.

6. A process according to Claim 1, wherein 1.0—5.0 parts by weight of the acetic acid is used per part by weight of the raw material dimethyl aromatic aldehyde.

7. A process according to Claim 1, wherein the oxidation is carried out under a pressure of 588—2942 K Pa (6—30 kg/cm²)gage.

8. A process according to Claim 1, wherein the oxidation is carried out for a residence time of 1—5 hours.

## Revendications

1. Un procédé pour la préparation de l'acide trimellitique et pyromellitique par oxydation à l'air en phase liquide d'au moins un composé parmi le 2,4-diméthylbenzaldéhyde, le 3,4-diméthylbenzaldéhyde et le 2,5-diméthylbenzaldéhyde dans l'acide acétique comme solvant en présence de cobalt, de manganèse, et de brome comme catalyseur, dans lequel l'amélioration comprend la réalisation de l'oxydation dans un réacteur à mélange complet à une concentration totale du cobalt et du manganèse de 0,05—0,5% en poids par rapport à l'acide acétique et une température de réaction de 180°—240°C.

2. Un procédé selon la revendication 1, dans lequel un rapport atomique du cobalt au managanèse est 1:6—4:1 et un rapport atomique du brome à la somme du cobalt et du manganèse est 0.8:1—2,5:1.

3. Un procédé selon la revendication, 2 dans lequel le rapport atomique du brome à la somme du cobalt et du manganèse est 2,0:1.

4. Un procédé selon la revendication 1, dans lequel le cobalt et le manganèse sont sous la forme de sels d'acides organiques, de sels d'acides inorganiques ou de complexes organiques et le brome est sous la forme de brome simple, de bromures inorganiques ou de bromures organiques.

5. Un procédé selon la revendication 1, dans lequel l'acide acétique est de l'acide acétique à 95%.

6. Un procédé selon la revendication 1, dans lequel on utilise 1,0—5,0 parties en poids d'acide acétique par partie en poids de la matière première aldéhyde aromatique diméthylique.

7. Un procédé selon la revendication 1, dans lequel l'oxydation est réalisée sous une pression manométrique de 588—2 942 K Pa (6—30 kg/cm²).

8. Un procédé selon la revendication 1, dans lequel l'oxydation est réalisée pendant une durée de 1—5 heures.

## Patentansprüche

1. Verfahren zum Erzeugen von Trimellitsäure oder Pyromellitsäure indem mindestens eine der Věrbindungen 2,4-Dimethylbenzaldehyd, 3,4 - Dimethylbenzaldehyd und 2,5-Dimethylbenzaldehyd in Essigsäure als Lösungsmittel in Anwesenheit von Kobalt, Mangan und Brom als Katalysator in flüssiger Phase mit Luft oxidiert wird, dadurch gekennzeichnet, daß die Oxidation in einem eine vollständige Vermischung bewirkenden Reaktionsgefäß bei einer auf die Essigsäure bezogen Gesamtkonzentration von 0,05 bis 0,5 Gew.% Kobalt und Mangan und bei einer Reaktionstemperatur von 180 bis 240°C durchgeführt wird.

2. Verfahren nach Anspruch 1, dadurch gekennzeichnet, daß das Atomverhältnis von Kobalt zu Mangan 1:6 bis 4:1 und das Atomverhältnis von Brom zu der Gesamtmenge von Kobalt und Mangan zwischen 0,8:1 und 2,5:1 beträgt.

3. Verfahren nach Anspruch 2, dadurch gekennzeichnet, daß das Atomverhältnis von Brom zu der Gesamtmenge von Kobalt und Mangan 2,0 bis 1 beträgt.

4. Verfahren nach Anspruch 1, dadurch gekennzeichnet, daß Kobalt und Mangan in Form von Salzen von organischen Säuren, Salzen von anorganischen Säuren oder organischen Komplexverbindungen verwendet werden und Brom in Form von elementarem Brom, anorganischen Bromiden oder organischen Bromiden verwendet wird.

5. Verfahren nach Anspruch 1, dadurch gekennzeichnet, daß als Essigsäure 95%ige Essigsäure verwendet wird.

6. Verfahren nach Anspruch 1, dadurch gekennzeichnet, daß die Essigsäure in einer Menge von 1,0 bis 5,0 Gew.% des als Ausgangsmaterial verwendeten Dimethylarom. Aldehyds verwendet wird.

7. Verfahren nach Anspruch 1, dadurch gekennzeichnet, daß die Oxidation unter einem Überdruck von 588 bis 2942 kPa durchgeführt wird.

8. Verfahren nach Anspruch 1, dadurch gekennzeichnet, daß die Oxidation mit einer Verweilzeit von 1 bis 5 Stunden durchgeführt wird.